(19) Europäisches Patentamt
European Patent Office
Office européen des brevets

(11) **EP 2 047 804 A1**

(12) **EUROPEAN PATENT APPLICATION**
published in accordance with Art. 153(4) EPC

(43) Date of publication:
**15.04.2009 Bulletin 2009/16**

(51) Int Cl.:
**A61B 8/08** (2006.01)

(21) Application number: **06782360.9**

(22) Date of filing: **04.08.2006**

(86) International application number:
**PCT/JP2006/315502**

(87) International publication number:
**WO 2008/015761 (07.02.2008 Gazette 2008/06)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicants:
  • **Shimadzu Corporation**
    **Kyoto-shi**
    **Kyoto 604-8511 (JP)**
  • **Media Cross Co., Ltd**
    **Shinjuku-ku**
    **Tokyo 160-0012 (JP)**

(72) Inventors:
  • **KATOH, Jun-ichi**
    **Nakagyo-ku, Kyoto 604-8511 (JP)**
  • **ITOH, Masao**
    **Shinjuku-ku, Tokyo 160-0012 (JP)**

(74) Representative: **Wilhelms · Kilian & Partner**
  **Patentanwälte**
  **Eduard-Schmid-Strasse 2**
  **81541 München (DE)**

(54) **ULTRASONOGRAPH**

(57)    The schedule memory unit 23 memorizes a time schedule set in advance for saving images. The avascularization unit 30 compresses the subject's brachial region to avascularize the brachial artery, releases the avascularization after a predetermined period of time, and simultaneously transmits a decompression start signal to the controller 17. The controller 17 stores an ultrasonic image in the image storage unit 22 at a point in time when a period of time specified by the time schedule has elapsed after the decompression start signal was received. The vascular diameter measuring unit 18 determines the vascular diameters based on the ultrasonic tomography images of a blood vessel at rest and after the release of the avascularization which are stored in the image storage unit 22. Based on those diameter values, the largest vascular diameter deducing unit 20 and the %FMD calculation unit 21 perform the deduction of the largest after-release vascular diameter and the calculation of the %FMD, respectively. Therefore, after the avascularization is started, it is possible to automatically perform the process from saving a measurement image to the calculation of the %FMD without the operator's aid. Accordingly, the operator's inconvenience in performing a %FED can be significantly decreased.

Fig. 2

START

S11 SETTING THE TIME SCHEDULE

S12 FIXING THE PROBE

S13 OBTAINMENT OF A RESTING VASCULAR IMAGE

S14 AVASCULARIZATION

S15 RELEASE

S16 STORING THE AFTER-RELEASE VASCULAR IMAGE

S17 MEASURING THE VASCULAR DIAMETER

S18 DEDUCING THE LARGEST VASCULAR DIAMETER

S19 CALCULATION OF %FMD

END

**Description**

TECHNICAL FIELD

The present invention pertains to an ultrasonograph, and particularly to an ultrasonograph preferably used for the measurement of a vascular diameter.

BACKGROUND ART

Since arteriosclerosis causes examples of heart disease such as angina pectoris and myocardial infarction, cerebral infarction, and other diseases, it is preferable to regularly perform an examination. One known method for noninvasively assessing the pliancy of a blood vessel for an early diagnosis of arteriosclerosis is a flow mediated dilation (FMD) test using an ultrasonograph (refer to Patent Document 1 for example).

A flow mediated dilation is a reaction in which an increase in blood flow generates a shear stress which acts on the vascular endothelium, causing nitric monoxide which is a vasodilating agent to generate from an endocapillary cell and dilate the vascular diameter. In a flow mediated dilation test, the dilation degree of the vascular diameter in the brachial region is measured when an antebrachial region has been avascularized for a given period of time and then quickly released, The %FMD which represents the dilation degree is defined with the following formula:

$$\%FMD = \frac{D_1 - D_0}{D_0} \times 100(\%) \qquad \ldots(1)$$

where, $D_0$ represents the largest vascular diameter at rest before the avascularization (which will be hereinafter called the "largest resting vascular diameter"), and $D_1$ represents the largest vascular diameter after the release of the avascularization (which will be hereinafter called the "largest after-release vascular diameter").

The flow mediated dilation test (which will be hereinafter called a "%FMD measurement") is performed as follows. First, an ultrasonic probe is applied to the brachial region of a subject, and a tomographic image of a brachial artery at rest is portrayed by an ultrasonic scan. The vascular tomographic image obtained by the ultrasonic scan is renewed at predetermined intervals to be displayed on the monitor as a moving image. Next, the antebrachial region of the subject is compressed with a cuff for avascularization for a given period of time, and after that, the tomographic image of the brachial artery after a quick release is take. The ultrasonic images taken in a series of imagings are saved by a video cassette recorder (VCR). After the imaging is finished, an operator plays the moving image recorded by the VCR and selects an image appropriate for the measurement of the largest resting vascular diameter and largest after-release vascular diameter. After that, the diameter of the blood vessel portrayed in each image is measured by an image analysis. With the largest resting vascular diameter $D_0$ and the largest after-release vascular diameter $D_1$ obtained by this operation, the %FMD is calculated based on the formula (1).

[Patent Document Japanese Unexamined Patent Application Publication No. 2003-180690 ([0002])

DISCLOSURE OF THE INVENTION

PROBLEMS TO BE SOLVED BY THE INVENTION

As previously described, a %FMD measurement is cumbersome in that, after the imaging is finished, an operator has to play the images recorded by a VCR and select an appropriate image for the measurement of the vascular diameter. In addition, in a %FMD measurement, only the images synchronized with the cardiac cycle's R-wave, i.e. the ultrasonic images obtained in a vasodilatory period, are generally used as a measurement object in order to exclude the influence of the heart throb on the vascular diameter's change. Accordingly, performing an imagine as previously described requires the following operation: detect a cardiac cycle by an electrocardiograph while simultaneously performing an ultrasonic scan, and after the imaging is finished, select an image appropriate for the measurement of the largest resting vascular diameter $D_0$ and the largest after-release vascular diameter $D_1$ while referring to the ultrasonic scan images and the electrocardiographic waveform recorded in the VCR. Thus, the image selection operation is troublesome.

In addition, in storing a series of images by the VCR, the avascularization release time (i.e. decompression start time) is not recorded. Therefore, in order to know how many seconds have elapsed from the release of the avascularization until the obtainment of the image selected for the measurement, the operator has to record, in imaging, the decompression start time and based on that, calculate the elapsed time since the initiation of the decompression.

Generally, in obtaining the largest after-release vascular diameter $D_1$, the vascular diameter at rest and those at a plurality of points in time after the avascularization release are measured, and the largest after-release vascular diameter $D_1$ is deduced based on the plural measurement values thus obtained. However, such deduction of the largest vascular diameter and the calculation of the %FMD after that are performed by a personal computer provided independently from the ultrasonograph, which leads to a low work efficiency.

The present invention has been accomplished to solve such problems, and the objective thereof is to provide an ultrasonograph capable of simply and easily performing a %FMD measurement.

MEANS FOR SOLVING THE PROBLEMS

To solve the previously described problems, the present invention provides an ultrasonograph including an ultrasonic image generator for transmitting and receiving an ultrasonic wave for a subject and generating ultrasonic images of the subject from obtained echo data, including: a schedule setting means for setting a time schedule which contains timings of storing the ultrasonic images; and an image storing means for storing each of the ultrasonic images as an image file at a point in time when a period of time specified by the schedule has elapsed after a predetermined trigger signal was generated.

The image storing means may preferably have a function of saving the ultrasonic image under a file name including a code indicating an elapsed time after the trigger signal is generated and before the image is stored.

As the trigger signal, a signal generated in response to the operation of a predetermined key, button, etc provided on the main body of the ultrasonograph can be used for example. In this case, when an operator operates the key or the like in accordance with the timing of the avascularization release by the cuff, an images storing in accordance with the time schedule is performed.

The ultrasonograph may include an automatic avascularization means for compressing a body surface of a subject to avascularize a blood vessel to be diagnosed, decompressing after a predetermined period of time to release the avascularization, and simultaneously providing a decompression start signal. In this case, the decompression start signal may be used as the trigger signal.

Preferably, the ultrasonograph according to the present invention may further include a cardiac cycle detector for detecting the cardiac cycle or the subject and transmitting a cardiac cycle signal synchronized with the cardiac cycle, and a generation of an image by the ultrasonic image generator or a storing of an image by the image storing means may be performed at timing synchronized with the cardiac cycle.

In addition, the ultrasonograph according to the present invention may preferably further include: a vascular diameter measurement means for measuring a vascular diameter based on an ultrasonic tomography image of a blood vessel stored in the image storing means; and a %FMD calculator for calculating a %FMD based on a vascular diameter at rest and a vascular diameter after a release of an avascularization measured by the vascular diameter measurement means.

EFFECTS OF THE INVENTION

With the ultrasonograph according to the present invention which has the aforementioned configuration, the storing of the ultrasonic tomography image (still image) is performed at a previously specified timing after the release of an avascularization. Therefore, the operator does not need to take time for the following conventional tasks: playing the images recorded in the VCR after the imaging is completed, and selecting images appropriate for the measurement of the largest resting vascular diameter and the largest after-release vascular diameter. In the case where a code indicating the elapsed time is included in the name of the image file, even in observing the image at a later time, it is possible to easily know in what situation the image was obtained, which serves the diagnosis.

In the case where the ultrasonograph includes the cardiac cycle detector and the generation or storing of an ultrasonic image is performed at a timing synchronized with the subject's cardiac cycle, the measurement of the vascular diameter can always be performed based on the ultrasonic image corresponding to the same cardiac phase, Hence, the influence of the heart throb on the vascular diameter's change can be exclude, which brings about a higher reliable diagnosis.

In the case where the ultrasonograph includes the automatic avascularization means as previously described and the decompression start signal provided from the automatic avascularilzation means is used as the trigger signal, it is possible to link the automatic avascularization means and the image storing means. Consequently, after the avascularization is started, the storing of the measurement images can be automatically performed without troubling the operator.

In the case where the ultrasonograph includes the vascular diameter measurement means and the %FMD calculator as previously described, after the avascularization is started, the following process can be automatically performed without the operator's aid: storing of the measurement image, measurement of the vascular diameter, and the calculation of the %FMD. Accordingly, a %FMD measurement which has been cumbersome can now be easily performed.

BRIEF DESCRIPTION OF THE DRAWINGS

Fig. 1 is a block diagram illustrating a schematic configuration of an ultrasonograph according to an embodiment of the present invention.
Fig. 2 is a flowchart illustrating the procedure of a %FMD measurement using the ultrasonograph according to the same embodiment.
Fig. 3 is a diagram illustrating an example of a time schedule setting window in the ultrasonograph according to the same embodiment.
Fig. 4 is a pattern diagram illustrating the state where an ultrasonic probe according to the same embodiment is applied to the subject's brachial region.
Fig. 5 is a diagram illustrating an example of an axial tomographic image of a brachial artery.

EXPLANATION OF NUMERALS

| | |
|---|---|
| 10 | Main Body of Ultrasonograph |
| 11 | Ultrasonic Probe |
| 12 | Transmission Controller |
| 13 | Ultrasonic Signal Processor |
| 14 | Display Processor |
| 15 | Monitor |
| 16 | Input Unit |
| 17 | Controller |
| 18 | Vascular Diameter Measuring Unit |
| 19 | Measurement Result Memory Unit |
| 20 | Largest Vascular Diameter Deducing Unit |
| 21 | %FMD Calculation Unit |
| 22 | Image Storage Unit |
| 23 | Schedule Memory Unit |
| 30 | Avascularization Unit |
| 31 | Cuff |
| 32 | Air Pipe |
| 33 | Press Pump |
| 34 | Avascularization Controller |
| 40 | Cardiac Cycle Detection Unit |
| 41 | Electrocardiographic Detection Sensor |
| 42 | Cardiac Cycle Signal Obtainment Unit |
| 70 | Brachial Artery |
| 71 | Intravascular Lumen |
| 72 | Vascular Wall |

BEST MODE FOR CARRYING OUT THE INVENTION

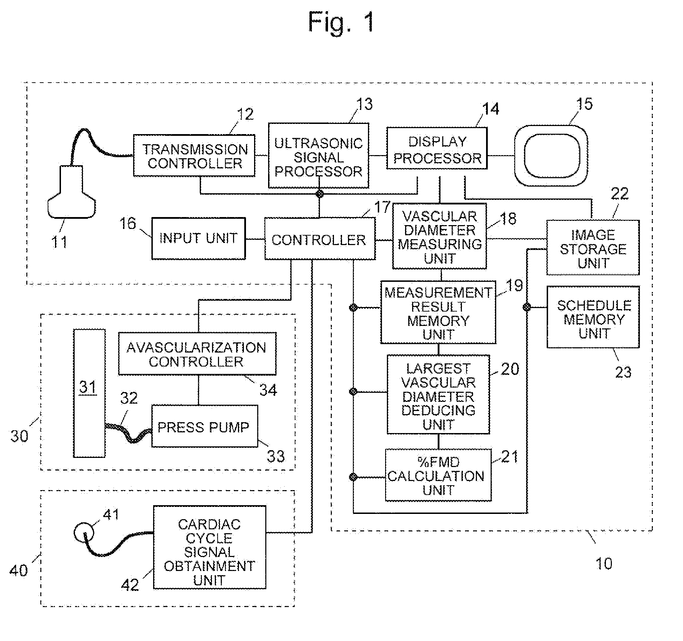

Hereinafter, the best mode for carrying out the present invention will be described using an embodiment. Fig. 1 illustrates a schematic configuration of an ultrasonograph according to the present embodiment.

An ultrasonic probe 11 transmits an ultrasonic wave to the inside of the body of a subject and simultaneously receives the ultrasonic wave reflected inside the subject's body to convert it into an electrical signal A transmission controller 12 controls the ultrasonic beam scan by the ultrasonic probe 11.

The electrical signal of the reflected ultrasonic wave provided by the ultrasonic probe 11 is fed to an ultrasonic signal processor 13. The ultrasonic signal processor 13 performs a data processing to the electrical signal appropriate for displaying the image, such as a phased summation, gain adjustment or logarithmic compression, After the processing, the data is delivered from the ultrasonic signal processor 13 to a display processor 14 In the display processor 14, an electrical signal to be displayed on a monitor 16 is created from the image data, and the electrical signal is displayed on the monitor 15. The operations described to this point are repeated at predetermined intervals, and the ultrasonic images are displayed on the monitor 15 as a moving image. The ultrasonic images may be saved in an image storage unit 22 composed of a storage device such as a hard disk in response to the operator's instruction. In addition, a time schedule which contains timings of storing images may be saved in a schedule memory unit 23, in which case the ultrasonic images can be automatically stored in the image storage unit 22 in accordance with the time schedule.

A vascular diameter measuring unit 18 performs the detection of a vascular wall and the measurement of the distance between the vascular walls by using a predetermined image analysis algorithm to the blood vessel's tomographic image stored in the image storage unit 22 in order to derive the vascular diameter. A measurement result memory unit 19 stores the measurement result by the vascular diameter measuring unit 18, and a largest vascular diameter deducing unit 20 deduces the largest after-release vascular diameter from a plurality of measurement values stored in the measurement result memory unit 19. Based on the values of the largest after-release vascular diameter $D_1$ and the largest resting vascular diameter $D_0$ thus obtained, the value of the %FMD is calculated in a %FMD calculation unit 21.

Each of the aforementioned units is controlled by a controller 17 which is composed of a central processing unit (CPU) or the like. To the controller 17, an input unit 16 is connected which is composed of a pointing device such as a mouse or trackball, keyboard, or other units for allowing the user to enter the setting, instruction, etc.

In addition, the controller 17 is connected to an avascularization unit 30 for avascularizing the subject's brachial artery and a cardiac cycle detection unit 40 for detecting the subject's cardiac cycle. The avascularization unit 30 has a cuff 31 which is wrapped around the subject's antebrachial region to be used, an air pipe 32 and a press pump 33 for pumping air into the cuff 31, and an avascularization controller 34 for controlling these units. The press pump 33 has an electromagnetic valve for discharging the air. When an avascularization is released, the electromagnetic valve is opened, and simultaneously a signal (which will be hereinafter called a "decompression start Signal") for indicating the initiation of the decompression of the cuff 31 is provided to the controller 17 in the main body 10 of the ultrasonograph. Additionally, an instruction from the operator can be given to the avascularization controller 34 through the input unit 16 provided in the main body 10 of the ultrasonograph.

The cardiac cycle detection unit 40 is composed of an electrocardiographic detection sensor 41 and a cardiac cycle signal obtainment unit 42. The electrocardiographic detection sensor 41 is a unit for detecting the subject's electrocardiogram and is attached to the body surface adjacent to the subject's heart. An existing electrocardiographic measurement sensor can be used as the electrocardiographic detection sensor 41. The electrocardiographic detection signal provided from the electrocardiographic detection sensor 41 is fed to the cardiac cycle signal obtainment unit 42. The cardiac cycle signal obtainment unit 42 obtains the cardiac cycle from the provided electrocardiographic detection signal and transmits a signal (cardiac cycle signal) to the controller 17 of the main body 10 of the ultrasonograph, In the present invention, the cardiac cycle signal may be, for example, a sine wave signal synchronized with the electrocardiogram, a pulsed signal transmitted at a given phase of the cardiac cycle, or other type of signal.

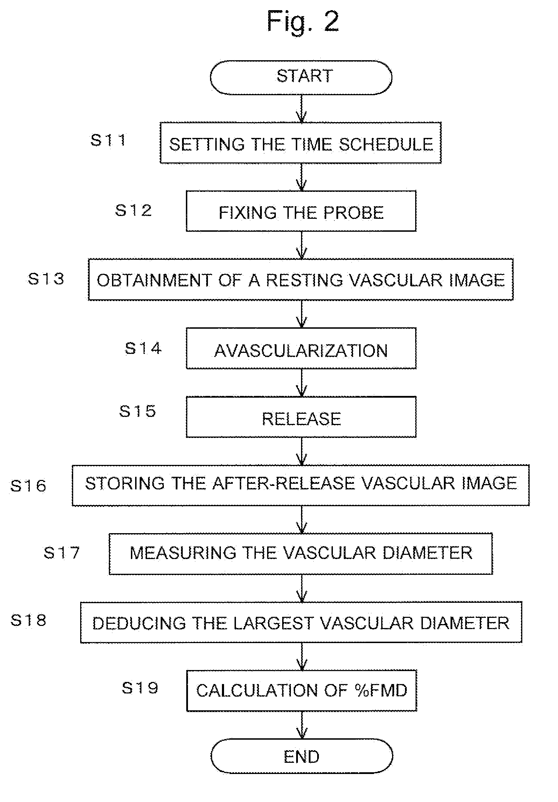

Next, a procedure of a %FMD measurement using the ultrasonograph according to the present embodiment will be described by using the flowchart of Fig. 2.

(1) Setting the Time Schedule (Step S11)

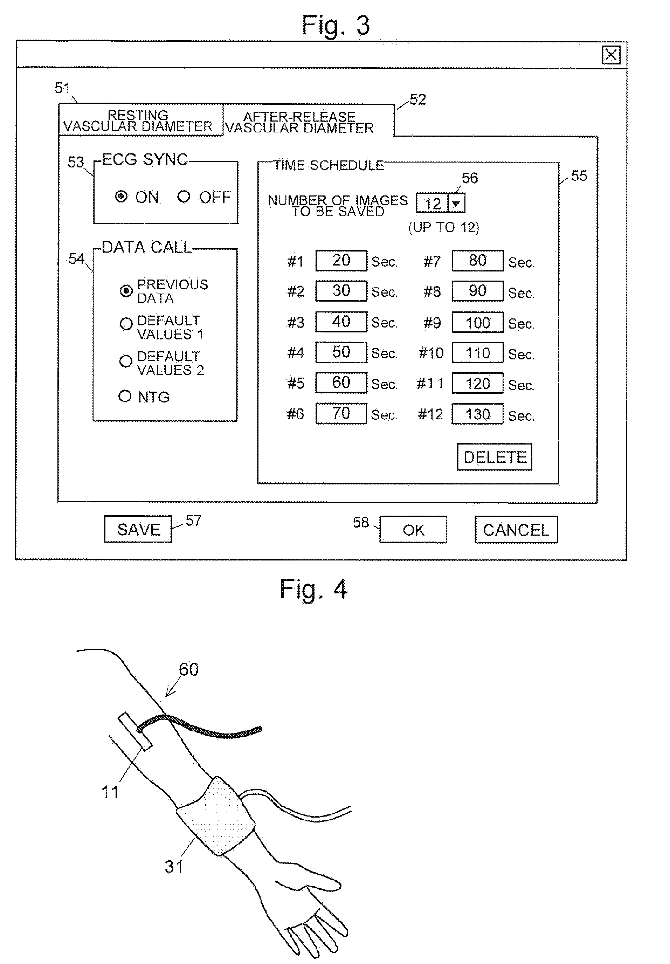

When the operator performs a predetermined operation through the input unit 16, a time schedule setting window as illustrated in Fig. 3 is displayed on the monitor 15, This time schedule setting window is used to set the conditions for saving the images for measuring the largest vascular diameter at rest (which will be hereinafter called a "resting vascular image") and the images for measuring the largest vascular diameter after the release (which will be hereinafter called an "after-release vascular image,") in the image storage unit 22. By clicking the tabs 51 and 52 provided inside the window, it is possible to switch the setting window for saving a resting vascular image and the setting window for saving an after-release vascular image. In the figure, the setting window for saving an after-release vascular image is illustrated. The setting window for saving an after-release vascular image is composed of an electrocardiogram (ECG) synchronization setting field 53, a data call selection field 54, and a time schedule setting field 55.

In the ECG synchronization setting field 53, it is possible to select whether or not to perform an image saving synchronized with the cardiac cycle signal by the electrocardiographic detection sensor 41. In the case where the "ON" option is selected, only an ultrasonic image at a predetermined cardiac phase (generally the R-wave) is stored in the image storage unit 22, and in the case where the "OFF" option is selected, an image saving is performed regardless of the cardiac phase. The time schedule setting window 55 is provided in order to set the timing of an image saving. The operator selects the number (up to 12) of images to be saved from the pull-down menu 56 and fills in the values of the fields #1 through #12 in accordance with the number of images to specify the period of time from the initiation of the decompression to the saving of the image. In the time schedule setting field 55, the numerical values can be entered through a keyboard or other device provided in the input unit 16. Alternatively, any one of the "previous data" (i.e. the most recently used time schedule), "default values 1", "default values 2", and "NTG" (i.e. the time schedule used in measuring the vascular diameter's rate of change after a nitroglycerin administration) may be selected in the data call selection field 54 to read out an existing time schedule. After filling in each field, the "SAVE" button 57 may be clicked to save these settings so that they can be called and used according to necessity in subsequent measurements. In the setting window for saving a resting vascular image, only an ECG synchronization setting field 53 which is the same as described earlier is provided.

When the input in the time schedule setting window is completed, and the operator clicks the "OK" button 58, the time schedule setting window is terminated and these settings are saved in the schedule memory unit 23.

(2) Fixing the Probe (Step S12)

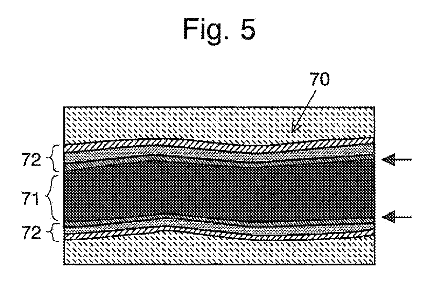

Next, the electrocardiographic detection sensor 41 is attached to the subject's chest region, the cuff 31 is wrapped around the antebrachial region, and the subject is kept at rest for fifteen minutes. Then, the probe 11 is applied to predetermined position of the brachial region 60 (Fig. 4). At this point in time, the probe 11 is set in such a manner that the array direction of the oscillator array (i.e. the scan direction of the ultrasonic beam) and the axial direction of the arm are parallel. Subsequently, the ultrasonic beam scan by the probe 11 is performed and an axial tomographic image of the brachial artery 70 as illustrated in Fig. 5 is portrayed on the monitor 15. The operator searches for the position where the diameter of the portrayed blood vessel 70 is largest by manually moving the position and angle of the probe 11 while looking at the image on the monitor 15, and fixes the probe 11 with a fixture (not shown) so that the probe will not move from the position.

(3) Saving a Resting Vascular Image (Step S13)

During the aforementioned state, ultrasonic images are taken. And then, when the operator performs a predetermined operation through the input unit 16, the controller 17 saves the images at the timing corresponding to the cardiac cycle signal transmitted by the cardiac cycle signal obtainment unit 42 (in the case where the ECG synchronization is in the "ON" state), That is, after the instruction by the operator, the images are saved at the timing corresponding to the first R-wave and stored in the images storage unit 22. In the case where the ECG synchronization is in the "OFF" state, the storing of the ultrasonic images is performed, regardless of the cardiac phase, at the point in time where the operator's instruction is received. The operator may perform the predetermined operation plural times to save a plurality of resting vascular images..

(3) Avascularization (Step S14) and Release (Step S15)

After the storing of the resting vascular image is completed, the operator performs a predetermined operation through the input unit 16 to initiate the avascularization of the brachial artery by the cuff 31. In the avascularization unit 30, the avascularization controller 34 controls the press pump 33 to pump air into the cuff 31 which is wrapped around the subject's antebrachial region and halts the air supply when the pressure reaches 250mmHg. Five minutes later, the avascularization controller 34 opens the electromagnetic valve provided in the press pump 33 in order to discharge the air to quickly reduce the pressure. Upon opening the electromagnetic valve, the avascularization controller 34 also transmits a decompression start signal to the controller 17 in the main body 10 of the ultrasonograph.

(5) Saving an After-Release Vascular Image (Step S16)

When the decompression start signal is fed to the controller 17, the controller 17 refers to the time schedule stored in the schedule memory unit 23, and saves an ultrasonic image in the image storage unit 22 as an image file at every point in time when the time that has elapsed since the reception of the decompression start signal reaches a predetermined period of time specified in the time schedule. For example, in the case where a time schedule as illustrated in Fig. 3 is set, the first R-wave synchronized image is saved twenty seconds after the reception of the decompression start signal, and subsequently eleven R-wave synchronized images are saved at ten second interval, Simultaneously, a portion of the file name of each generated image file is set to include a code indicating the timing when the image has been saved (i.e. the elapsed time from the initiation of the decompression). The ultrasonic scan is terminated when all the ultrasonic images have been saved at all the timings specified by the time schedule (in this embodiment, at twelve points corresponding to the elapsed times #1 through #12). After receiving the decompression start signal, preferably, the number of seconds that have elapsed after the initiation of the decompression may be superimposed on an ultrasonic image, and the elapsed time may be saved with the ultrasonic image.

(6) Measuring a Vascular Diameter (Step S17)

After the imaging is completed, each image file stored in the image storage unit 22 is transmitted to the vascular diameter measuring unit 18. In the vascular diameter measuring unit 18, for the image data (or brightness value of each picture element) of the resting vascular image and after-release vascular image stored in the image storage unit 22, the boundary positions (which are indicated by the arrows in Fig. 5) between the intravascular lumen 71 and two vascular walls 72 (one wall closer to the skin and the other father from it) are detected based on the brightness distribution. The distance between the two boundary positions is measured as the vascular diameter. The measurement value obtained for each image is stored in the measurement result memory unit 19. For such a detection of the vascular walls and measurement of the vascular diameter, a conventionally known algorithm can be used for example.

(7) Deduction of the Largest after-release Vascular Diameter (Step S18) and Calculation of the %FMD (Step S19)

After the vascular diameter measurement for all the images is completed, the measurement values for the vascular diameter at rest and at each of the elapsed times #1 through #12 after the avascularization release are read out from the measurement result memory unit 19 to the largest vascular diameter deducing unit 20. Then, based on these values, the value of the largest after-release vascular diameter $D_1$ is deduced by a regression analysis. After that, using the value of $D_1$ and the value of the largest resting vascular diameter $D_0$ stored in the measurement result memory unit, the %FMD is calculated by the aforementioned formula (1). The obtained result is displayed on the monitor 15.

As just described, with the ultrasonograph according to the present embodiment, after a time schedule is set in advance, the resting vascular images are stored, and the avascularization by the cuff is started, the following process is automatically performed without the operator's aid: the saving of the after-release vascular images, measurement of the vascular diameter, deduction of the largest after-release vascular diameter, and calculation of the %FMD. Accordingly, the operator's inconvenience in performing a %FMD measurement is decreased and the measurement can be simply and easily performed, In addition, each image file which is stored in the image storage unit is given a file name including a code that indicates how many seconds has elapsed from the initiation of the decompression when the image concerted has been obtained. Therefore, when looking at the image file at a later date, the operator can know at a glance in what situation the image has been taken.

The best mode for carrying out the present invention has been described so far using an embodiment. However, it should be noted that the present invention is not limited to the previously described embodiment, and a variety of modifications are allowed within the spirit of the present invention. For example, the ultrasonograph according to the present invention may preferably be configured as a single apparatus that can independently perform the operations from taking an ultrasonic image to calculating the %FMD as in the aforementioned embodiment: however, this configuration is merely an option. For example, the ultrasonograph may be designed to perform Steps S 11 through S17 yet leave Steps S18 and S19 to be performed by a personal computer or other devices provided separately from the ultrasonograph.

The ultrasonograph according to the present invention may be used for a %FMD measurement as previously described, and in addiction, it may be used for the measurement of a stiffness parameter $\beta$, which is an index of a degree of vascular sclerosis, or other measurements. In such a case, since a blood pressure measurement must be performed simultaneously with an ultrasonic scan, an avascularization unit with a function of measuring the blood pressure should be used.

**Claims**

1. An ultrasonograph including an ultrasonic image generator for transmitting and receiving an ultrasonic wave for a subject and generating ultrasonic images of the subject from obtained echo data, comprising:

   a) a schedule setting means for setting a time schedule which contains timings of storing the ultrasonic images; and
   b) an image storing means for storing each of the ultrasonic images as an image file at a point in time when a period of time specified by the time schedule has elapsed after a predetermined trigger signal was generated.

2. The ultrasonograph according to claim 1, wherein the image storing means saves the ultrasonic image under a file name including a code indicating an elapsed time after the trigger signal is generated and before the image is stored.

3. The ultrasonograph according to either claim 1 or 2, further comprising

   c) an automatic avascularization means for compressing a body surface of a subject to avascularize a blood vessel to be diagnosed, decompressing after a predetermined period of time to release the avascularization, and simultaneously providing a decompression start signal, wherein the image storing means operates with the decompression start signal as the trigger signal.

4. The ultrasonograph according to any one of claim 1 through 3, further comprising

   d) a cardiac cycle detector for detecting a cardiac cycle of the subject and transmitting a cardiac cycle signal synchronized with the cardiac cycle, wherein
   a generation of an image by the ultrasonic image generator or a storing of an image by the image storing means is performed at a timing synchronized with the cardiac cycle.

5. The ultrasonograph according to any one of claims 1 through 4, further comprising:

   e) a vascular diameter measurement means for measuring a vascular diameter based on an ultrasonic tomography image of a blood vessel stored by the image storing means; and
f) a %FMD calculator for calculating a %FMD based on a vascular diameter at rest and a vascular diameter after a release of an avascularization measured by the vascular diameter measurement means.

# Fig. 1

# Fig. 2

```
                    ┌─────────────┐
                    │    START    │
                    └──────┬──────┘
                           ↓
S11        ┌────────────────────────────────┐
           │    SETTING THE TIME SCHEDULE    │
           └────────────────┬───────────────┘
                            ↓
S12           ┌──────────────────────────┐
              │      FIXING THE PROBE     │
              └─────────────┬────────────┘
                            ↓
S13    ┌──────────────────────────────────────────┐
       │  OBTAINMENT OF A RESTING VASCULAR IMAGE   │
       └─────────────────────┬────────────────────┘
                             ↓
S14          ┌───────────────────────────────┐
             │        AVASCULARIZATION        │
             └───────────────┬───────────────┘
                             ↓
S15           ┌──────────────────────────┐
              │         RELEASE           │
              └─────────────┬────────────┘
                            ↓
S16    ┌──────────────────────────────────────────────┐
       │   STORING THE AFTER-RELEASE VASCULAR IMAGE    │
       └─────────────────────┬────────────────────────┘
                             ↓
S17      ┌─────────────────────────────────────────┐
         │     MEASURING THE VASCULAR DIAMETER      │
         └───────────────────┬─────────────────────┘
                             ↓
S18    ┌──────────────────────────────────────────────┐
       │    DEDUCING THE LARGEST VASCULAR DIAMETER     │
       └─────────────────────┬────────────────────────┘
                             ↓
S19         ┌─────────────────────────────────┐
            │      CALCULATION OF %FMD         │
            └────────────────┬────────────────┘
                             ↓
                    ┌─────────────┐
                    │     END     │
                    └─────────────┘
```

9

# Fig. 3

| RESTING VASCULAR DIAMETER | AFTER-RELEASE VASCULAR DIAMETER |

ECG SYNC
◉ ON  ○ OFF

DATA CALL
◉ PREVIOUS DATA
○ DEFAULT VALUES 1
○ DEFAULT VALUES 2
○ NTG

TIME SCHEDULE

NUMBER OF IMAGES TO BE SAVED [ 12 ▼ ]
(UP TO 12)

#1 [ 20 ] Sec.    #7 [ 80 ] Sec.
#2 [ 30 ] Sec.    #8 [ 90 ] Sec.
#3 [ 40 ] Sec.    #9 [ 100 ] Sec.
#4 [ 50 ] Sec.    #10 [ 110 ] Sec.
#5 [ 60 ] Sec.    #11 [ 120 ] Sec.
#6 [ 70 ] Sec.    #12 [ 130 ] Sec.

[ DELETE ]

[ SAVE ]    [ OK ]    [ CANCEL ]

# Fig. 4

## Fig. 5

## INTERNATIONAL SEARCH REPORT

| International application No. |
| --- |
| PCT/JP2006/315502 |

A. CLASSIFICATION OF SUBJECT MATTER
*A61B8/08*(2006.01)i

According to International Patent Classification (IPC) or to both national classification and IPC

B. FIELDS SEARCHED

Minimum documentation searched (classification system followed by classification symbols)
A61B8/08

Documentation searched other than minimum documentation to the extent that such documents are included in the fields searched
Jitsuyo Shinan Koho          1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)
UTILITY MODEL FILE(PATOLIS), PATENT FILE(PATOLIS)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | JP 2005-28123 A (Saraya Co., Ltd., Media Cross Co., Ltd.), 03 February, 2005 (03.02.05), Particularly, Par. Nos. [0148], [0149] (Family: none) | 1,3,5<br>4<br>2 |
| D,Y<br>D,A | JP 2003-180690 A (Media Cross Co., Ltd., Softmedical Co., Ltd.), 02 July, 2003 (02.07.03), Particularly, Par. No. [0010] (Family: none) | 4<br>1-3,5 |
| A | JP 11-85772 A (Rigaku Denki Co., Ltd.), 30 March, 1999 (30.03.99), Full text; all drawings (Family: none) | 2 |

☐ Further documents are listed in the continuation of Box C.      ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 18 October, 2006 (18.10.06) | 24 October, 2006 (24.10.06) |

| Name and mailing address of the ISA/<br>   Japanese Patent Office | Authorized officer |
| --- | --- |
| Facsimile No. | Telephone No. |

Form PCT/ISA/210 (second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003180690 A